# NOUVEAU FASCICULE DE BREVET EUROPEEN

(11) **EP 2 162 116 B2**
(45) Date de publication et mention de la décision concernant l'opposition: **14.08.2019**
(45) Mention de la délivrance du brevet: 06.05.2015
(21) Numéro de dépôt: 08805843.3
(22) Date de dépôt: 23.05.2008
(51) Int. Cl.: A61K 9/00, A61K 9/107, A61K 47/10, A61K 47/34, A61K 47/36, A61K 47/38, A61K 47/44, A61Q 19/00, A61K 8/06, A61K 8/34, A61K 8/49, A61K 8/81, A61K 8/92

(54) **VEHICULE SOUS FORME D'UNE EMULSION HUILE-DANS-EAU NOTAMMENT DESTINE A UNE UTILISATION OPTALMIQUE OU DERMOCOSMETIQUE**
MITTEL IN FORM EINER WASSER-IN-ÖL-EMULSION, INSBESONDERE ZUR OPHTHALMISCHEN ODER HAUTKOSMETISCHEN VERWENDUNG
VEHICLE IN THE FORM OF A WATER-IN-OIL EMULSION IN PARTICULAR FOR OPHTHALMIC OR DERMOCOSMETIC USE

(30) Priorité: 29.05.2007 FR 0703796
(43) Date de publication de la demande: 17.03.2010
(73) Titulaire: Galderma Research & Development SNC, 06410 Biot (FR)
(72) Inventeur: SARRAZIN, Christian, F-06580 Pegomas (FR); DO, Marina, F-06500 Menton (FR); BOIX, Michèle, F-06340 Canthron (FR)
(74) Mandataire: Nederlandsch Octrooibureau
(86) Numéro de dépôt international: PCT/FR2008/050899
(87) Numéro de publication internationale: WO 2008/145943

(56) Documents cités:
- EP-A- 0 521 799
- EP-A- 1 095 659
- EP-A- 1 681 059
- EP-A2- 1 275 376
- WO-A2-2008/012367
- WO-A2-2008/012367
- DE-A1- 19 511 322
- US-A- 5 474 979
- US-A1- 2007 218 007
- VALENTA C ET AL: "The use of polymers for dermal and transdermal delivery" EUROPEAN JOURNAL OF PHARMACEUTICS AND BIOPHARMACEUTICS, ELSEVIER SCIENCE PUBLISHERS B.V., AMSTERDAM, NL, vol. 58, no. 2, septembre 2004 (2004-09), pages 279-289, XP004526312 ISSN: 0939-6411

## Description

La présente invention se rapporte à une émulsion de type huile-dans-eau contenant une quantité très faible d'agent tensioactif selon les revendications ci-jointes, de sorte que l'émulsion est stable, bien tolérée et non irritante.

La présente invention se rapporte également à l'utilisation d'une telle invention comme base pour des compositions destinées à être appliquées sur des tissus sensibles du corps humain ou animal, en particulier dans des compositions ophtalmiques, dermatologiques et dermocosmétiques.

Une émulsion est le mélange de deux liquides non miscibles sous l'effet d'une agitation mécanique formant un système dispersé. Cette dispersion subsiste tant que dure l'agitation mais dès que cette dernière cesse, il y a coalescence des globules et les liquides se séparent.

La formulation d'une émulsion vise à apporter au mélange le ou les produits qui vont permettre ou faciliter la stabilisation du système. Ce ou ces produits sont donc essentiels à la formulation et la conservation en terme de stabilité physico-chimique d'une émulsion ; ils sont appelés émulsifiants, surfactants, émulgateurs agents mouillants ou encore tensioactifs et doivent être incorporés en une quantité relativement importante, de l'ordre de 1 à 2% dans le domaine ophtalmique.

Or les tensioactifs sont connus pour leur caractère irritant, ce qui limite fortement leur utilisation dans les domaines ophtalmiques et dermocosmétiques. Ainsi, dans le cadre de traitements à long terme en ophtalmologie par exemple, l'administration répétée de préparations sur la surface cornéenne entraîne des effets délétères, allergisants et toxiques (cas des traitements de la sécheresse oculaire, du glaucome...) pouvant aller d'une irritation simple à des altérations tissulaires sérieuses et susceptibles de compromettre le suivi du traitement par le patient.

De nombreux documents font largement état des effets cytotoxiques cornéens sur des modèles *in vitro* de composés tensioactifs reconnus, notamment le polysorbate 80.

Des effets indésirables du même type liés à l'utilisation de tensioactifs sont également observés dans le cadre d'applications dermiques.

Le problème technique que la présente invention cherche à résoudre consiste ainsi à trouver un compromis satisfaisant entre stabilité de l'émulsion et tolérance tissulaire.

Pour surmonter ce problème, l'homme du métier a cherché à supprimer l'utilisation de composés tensioactifs, dans de telles compositions, en les stabilisant à l'aide notamment d'agents viscosants.

Ainsi par exemple, la demande de brevet européen EP 1336404 décrit une composition lubrifiante fluide mimant le film lacrymal, qui est exempte d'agent tensioactif, et se présente sous forme d'une émulsion, dans laquelle la phase aqueuse contient au moins un agent de viscosité hydrosoluble et la phase huileuse qui représente 1,5 à 13% en poids de cette composition contient 1 à 6% en poids d'au moins un agent de viscosité liposoluble.

Néanmoins, cette composition utilise à titre de viscosant un composé tel que le monostéarate de glycérol, qui est par ailleurs connu de l'homme du métier pour ses propriétés émulsifiantes. Il s'agit d'un composé ayant une consistance de type semi-solide à température ambiante, conférant ainsi à la composition qui le contient une viscosité importante.

De la même façon, la demande de brevet internationale WO 96/37180 décrit des compositions galéniques utilisables en dermatologie et en cosmétologie de type pseudo-émulsions ne faisant pas intervenir de tensioactifs mais jouant sur l'épaississement des phases lipidique et aqueuse par l'emploi de monostéarate de glycérol.

Par ailleurs, le brevet européen EP 1 275 376, décrit une émulsion-gel consistant en une phase huileuse émulsionnée dans une solution aqueuse de polyvinylpyrrolidone ou de polyvinylalcohol ou de dextrane ou de dérivé de cellulose. La phase huileuse est immédiatement introduite dans la phase aqueuse préalablement gélifiée, de manière à bloquer et/ou stabiliser les globules lipidiques formés. L'alcool polyvinylique utilisé est de type totalement hydrolysé et les concentrations mises en oeuvre sont comprises entre 0.1 et 10%, et préférentiellement entre 0.3 et 1.5%.

Un but de la présente invention est donc de proposer un véhicule du type précité qui n'incorpore que de très faibles quantités d'agent tensioactif tout en conservant les caractéristiques généralement admises pour les émulsions classiques qui en contiennent largement plus, en particulier une taille de globules lipidiques faible de l'ordre de 2 à 50 µm. Les émulsions les plus fines favorisent la biodisponibilité des actifs véhiculés et sont plus stables à la conservation.

Pour cela, il est proposé un véhicule sous forme d'émulsion huile-dans-eau (H/E) comportant au moins une phase aqueuse (I) et au moins une phase huileuse (II), ladite phase aqueuse (I), comportant au moins un tensioactif hydrosoluble et au moins un agent viscosant hydrosoluble.

Selon l'invention, ledit au moins un tensioactif hydrosoluble est un alcool polyvinylique et représente 0,03 % en poids du poids total de véhicule.

Le véhicule selon l'invention est stable, bien toléré et non irritant.

En-dessous de 0,01% de tensioactif dans la phase aqueuse du véhicule selon l'invention, celui-ci présente l'inconvénient de ne pas contenir suffisamment de tensioactif pour permettre de réaliser une émulsion de stabilité optimale ainsi qu'une finesse en termes de taille de globules.

Au-dessus de 0,1% de tensioactif dans la phase aqueuse du véhicule selon l'invention, celui-ci présente l'inconvénient de contenir une quantité trop importante de tensioactif incompatible avec une bonne tolérance tissulaire, particulièrement pour des compositions contenant beaucoup d'huile.

A titre d'exemple d'agent tensioactif hydrosoluble utilisable dans le véhicule, on peut notamment citer les polysorbates, les lécithines, les polyéthylènes glycol et leurs dérivés, le polyoxyethylene-40-stéarate, les esters de sorbitanne, les copolymères polyoxyéthylène-polyoxypropylène, et les alcools polyvinyliques.

Selon l'invention, on utilise un alcool polyvinylique (PVA), et de manière davantage préféré un alcool polyvinylique partiellement hydrolysé, qui est largement utilisé dans les domaines pharmaceutique et cosmétique.

Par ailleurs, à titre d'agent viscosant hydrosoluble utilisable dans le véhicule selon l'invention, on peut citer les polymères acryliques ou carbomères, les gommes xanthanes, les carraghénanes, les alginates, la cellulose et ses dérivés.

Ainsi, on peut avantageusement utiliser à titre d'agent viscosant hydrosoluble un acide polyacrylique, et de préférence un acide polyacrylique réticulé, tel que par exemple celui commercialisé par la société NOVEON sous la dénomination commerciale Carbopol^{®} 980 NF. Un tel polymère a la propriété d'augmenter considérablement la viscosité de l'émulsion jusqu'à ce que celle-ci ait notamment la consistance d'un gel.

De même, on peut utiliser à titre d'agent viscosant hydrosoluble un copolymère acrylate/acrylate d'alkyle en C₁₀-C₃₀, tel que par exemple celui commercialisé par la société NOVEON sous la dénomination commerciale PEMULEN ^{®}.

La phase huileuse du véhicule selon l'invention est maintenant décrite plus en détail.

La phase huileuse du véhicule selon l'invention peut contenir une ou plusieurs huiles choisies parmi les huiles végétales, les huiles minérales, les huiles siliconées, les huiles de synthèse et les huiles fluorées.

A titre d'huile de synthèse utilisable selon la présente invention, on peut notamment citer le myristate d'isopropyle.

Comme huiles végétales utilisables selon l'invention, on peut citer les huiles de jojoba, avocat, sésame, tournesol, maïs, soja, carthame, pépins de raisin, olive, amande douce, ricin, moringa, coco, palme, bourrache, colza, germe de blé, lin, onagre, argan, calendula et coton.

Les huiles végétales préférées selon l'invention sont l'huile d'amande douce et l'huile de tournesol.

Comme huiles minérales utilisables selon l'invention, on peut citer les huiles de paraffine, et de préférence les huiles de paraffine liquide.

Le véhicule selon l'invention peut en outre comprendre un ou plusieurs additifs compatibles, qui ne modifient pas les caractéristiques propres aux émulsions.

A titre d'additifs compatibles avec un usage ophtalmique ou dermocosmétique, on peut citer par exemple les agents conservateurs, les antioxydants, les isotonisants, les agents chélatants, les tampons, les polymères, les charges, les gélifiants hydrophiles ou lipophiles, les filtres hydrophiles, et les absorbeurs d'odeur.

Bien entendu, l'homme de l'art veillera à choisir le ou les éventuels adjuvants à ajouter aux compositions selon l'invention, ainsi que leur concentration, de manière que les propriétés avantageuses attachées intrinsèquement aux compositions conformes à l'invention ne soient pas, ou substantiellement pas altérées par l'addition envisagée.

En particulier, ces adjuvants ne devront pas nuire aux propriétés de la composition selon l'invention, c'est-à-dire une bonne tolérance et l'absence d'irritabilité de l'oeil ou de la peau, en fonction du mode d'application envisagé.

Un isotonisant préféré est la glycérine.

Si le véhicule selon l'invention contient à titre d'isotonisant de la glycérine, sa quantité sera avantageusement ajustée de façon à obtenir une osmolalité adéquate avec une administration oculaire.

Avantageusement, la phase huileuse représente 0,5% à 5 % en poids, et de préférence environ 1,5% en poids du poids total du véhicule.

Selon un premier mode de réalisation spécifique de l'invention, la phase huileuse représente 0,5% à 3% en poids, de préférence environ 1,5% en poids du poids total du véhicule.

Si l'on ajoute à un tel véhicule un isotonisant tel que la glycérine, on obtient un véhicule qui peut directement être utilisé à titre de produit ophtalmique pour hydrater l'oeil (larmes artificielles) et/ou pour le traitement. Un tel véhicule peut également être utilisé comme base dans une composition ophtalmique.

Aussi, la présente invention a-t-elle encore pour objet une composition ophtalmique se présentant sous forme d'une émulsion huile-dans-eau, qui comprend :
- un véhicule conforme à ce premier mode de réalisation de l'invention, et
- au moins un actif à usage ophtalmique.

L'actif à usage ophtalmique peut être par exemple choisi parmi les antiseptiques, les antibiotiques, les anti-viraux, les anti-inflammatoires, les anti-allergiques, les anti-glaucomateux, les vasoconstricteurs, les anesthésiques, les cicatrisants, les mydriatiques, les myotiques, les produits de suppléance lacrymale, les produits pour le traitement de la cataracte, les produits pour le traitement des affections dégénératives rétiniennes et les produits de diagnostic.

L'actif à usage ophtalmique utilisable dans la composition selon l'invention peut être hydrosoluble et/ou liposoluble.

Comme actifs ophtalmiques liposolubles utilisables dans les compositions ophtalmiques selon l'invention, on peut par exemple citer la vitamine A, la cyclosporine A.

Comme actifs ophtalmiques hydrosolubles utilisables dans la composition ophtalmique selon l'invention, on peut par exemple citer la vitamine B12, utilisée en raison de ses propriétés cicatrisantes.

De façon connue, la composition de l'invention peut contenir également les adjuvants habituels dans le domaine ophtalmique, dans la mesure où l'adjuvant n'altère pas les propriétés recherchées pour la composition ophtalmique selon l'invention.

Selon un deuxième mode de réalisation spécifique de l'invention, la phase huileuse représente 1 à 5% en poids du poids total du véhicule selon l'invention. Dans ce cas, ce véhicule peut être utilisé comme base dans une composition cosmétique et/ou dermocosmétique, et dermatologique notamment destinée au traitement des zones sensibles du visage, par exemple autour des yeux.

Aussi, la présente invention a-t-elle encore pour objet une composition cosmétique et/ou dermocosmétique et dermatologique comprenant :
- un véhicule selon l'invention comprenant 1 à 5% en poids de phase huileuse par rapport au poids total du véhicule, et
- au moins un actif à usage cosmétique et/ou dermatologique.

L'actif à usage cosmétique et/ou dermatologique peut être également hydrosoluble et/ou liposoluble.

Comme actifs à usage dermatologique, on peut citer par exemple les corticoïdes, les antibiotiques, les antifongiques, les antiseptiques, les antiparasitaires, les antiherpétiques, les antiacnéiques, les antiprurigineux, les kératolitiques, les produits pour le traitement du psoriasis, les produits pour le traitement de la dermatite atopique.

Comme actifs hydrosolubles à usage cosmétique et/ou dermocosmétique utilisables selon l'invention, on peut notamment citer les protéines, les acides aminés, les polyols, l'urée, les sucres et les dérivés du sucre, les vitamines hydrosolubles, les extraits végétaux et les hydroxyacides.

Une vitamine hydrosoluble particulièrement préférée pour la composition cosmétique et/ou dermatologique selon l'invention est la vitamine B12, qui outre ses propriétés cicatrisantes, présente l'avantage de conférer à la composition selon l'invention une coloration esthétiquement attractive d'un point de vue cosmétique.

Comme actifs liposolubles à usage cosmétique et/ou dermocosmétique utilisables selon l'invention, on peut notamment citer les filtres UVA et UVB, et les vitamines liposolubles telles que le rétinol (vitamine A) et ses dérivés et le tocophérol (vitamine E) et ses dérivés.

La présente invention concerne également un procédé de préparation d'un véhicule, comprenant les étapes successives suivantes:
a) préparer une première émulsion huile-dans-eau en introduisant dans un réacteur 0,5% à 5% en poids d'au moins une huile par rapport au poids total du véhicule, puis ajouter une solution aqueuse d'alcool polyvinylique comprenant 1,5% en poids d'alcool polyvinylique en un volume tel que le rapport volumique de la phase aqueuse sur la phase huileuse est d'au moins 2/3 et préférentiellement 3/2,
b) préparation d'une solution aqueuse d'agent viscosant hydrosoluble, dont la concentration dépendra du viscosant utilisé et de l'application visée,
c) introduction de la première émulsion dans la solution aqueuse d'agent viscosant hydrosoluble, pour obtenir une deuxième émulsion comprenant une phase aqueuse et une phase huileuse, la quantité de première émulsion introduite dans la solution aqueuse d'agent viscosant hydrosoluble étant telle que l'alcool polyvinylique hydrosoluble représente 0,03% en poids du véhicule,

Le tensioactif et l'agent viscosant hydrosolubles sont tels que ceux décrits ci-dessus.

De préférence, on introduit dans la solution aqueuse d'agent viscosant une solution aqueuse d'agent isotonisant, de préférence la glycérine, à raison d'environ 2,3%. L'introduction de cette solution aqueuse d'agent isotonisant permet d'obtenir une osmolalité de l'ordre de 270 mOsm/kg compatible avec une application ophtalmique.

L'invention sera maintenant illustrée à l'aide des exemples non-limitatifs suivants. Les quantités sont indiquées en pourcentage en poids sauf indication contraire.

### EXEMPLES

### Produits

▪ Composants de la phase huileuse
   - huile végétale : huile de tournesol ou huile d'amande douce
▪ Composants de la phase aqueuse
   - eau purifiée
   - soude
   - agents tensioactifs hydrosolubles : alcool polyvinylique (PVA), polysorbate 80
   - agents viscosants hydrosolubles : acide polyacrylique réticulé, commercialisé par la société NOVEON sous la dénomination commerciale CARBOPOL(R) ; copolymère acrylate/acrylate d'alkyle, commercialisé par la société NOVEON sous la dénomination commerciale PEMULEN ®
   - isotonisant : glycérine

### EXEMPLE 1

On a préparé un premier exemple de véhicule V1 selon l'invention, comme suit.

On réalise tout d'abord une première émulsion (H/E) par dispersion d'huile dans un volume de 20 mL de solution aqueuse de PVA à 1,5% massique. L'introduction de l'huile dans la solution aqueuse de PVA est faite sous forte agitation (Ultra-turrax, 1 minute).

Par ailleurs, on a introduit 23 g de glycérine dans environ 600 mL d'une solution aqueuse de copolymère acrylate/acrylate d'alkyle sous agitation magnétique, pour obtenir une solution monophasique de glycérine et de copolymère acrylate/acrylate d'alkyle.

On introduit ensuite la première émulsion (H/E) à base de PVA dans la solution monophasique de glycérine et de copolymère acrylate/acrylate d'alkyle sous agitation magnétique pour obtenir l'émulsion finale constituant le véhicule 1 selon l'invention. Le pH est ensuite ajusté entre 6 et 7 avec de la soude et le volume final est ajusté avec de l'eau.

La composition E1 selon l'invention est présentée dans le tableau 1 ci-après.

### EXEMPLE 2

On a préparé, de la même manière qu'à l'exemple 1, un deuxième exemple E2 de composition selon l'invention, en remplaçant le copolymère acrylate/acrylate d'alkyle par un acide polyacrylique réticulé.

La composition du véhicule E2 selon l'invention est également présentée dans le tableau 1 ci-après.

### EXEMPLE COMPARATIF EC1

On a préparé un premier exemple de composition témoin similaire à celle de l'exemple E1 mais sans tensioactif. Pour cela, l'huile a été préalablement émulsionnée dans une fraction de solution de copolymère acrylate/acrylate d'alkyle.

La composition du véhicule témoin EC1 est présentée dans le tableau 1 ci-après.

### EXEMPLE COMPARATIF EC2

On a préparé un deuxième cas de composition témoin correspondant à celle de l'exemple E2 mais sans tensioactif. Pour pouvoir faire cet exemple comparatif qui ne contient pas de tensioactif, l'huile a été préalablement émulsionnée dans une fraction de la solution d'acide polyacrylique réticulé.

La composition du véhicule témoin EC2 est également présentée dans le tableau 1 ci-après.

### EXEMPLE COMPARATIF EC3

On a préparé un troisième cas de composition témoin correspondant à celle de l'exemple E1 mais en remplaçant la solution de PVA par une solution de polysorbate 80, de manière à obtenir une concentration finale de tensioactif de 1% correspondant à la concentration de tensioactif classiquement utilisée dans les émulsions connues de l'art antérieur.

La composition du véhicule témoin EC3 est également présentée dans le tableau 1 ci-après.

### EXEMPLE COMPARATIF EC4

On a préparé un quatrième cas de composition témoin correspondant à celle de l'exemple E1 mais en augmentant la concentration de la solution de PVA de manière à avoir une concentration finale de tensioactif de 1% correspondant à la concentration de tensioactif classiquement utilisée dans les émulsions de l'art antérieur.

La composition du véhicule témoin EC4 est également présentée dans le tableau 1 ci-après.

**Tableau 1**

| Composition (en g/pour 100g de véhicule) | E1 (en g/pour 100g de véhicule) | E2 (en g/pour 100g de véhicule) | EC1 (en g/pour 100g de véhicule) | EC2 (en g/pour 100g de véhicule) | EC3 (en g/pour 100g de véhicule) | EC4 (en g/pour 100g de véhicule) |
|---|---|---|---|---|---|---|
| - PVA | 0,03 | 0,03 | - | - | - | 1 |
| - polysorbate 80 | - | - | - | - | 1 | - |
| - glycérine | 2,3 | 2,3 | 2,3 | 2,3 | 2,3 | 2,3 |
| - acide polyacrylique réticulé | - | 0,25 | - | 0,25 | - | - |
| - copolymère acrylate/acrylate d'alkyle | 0,05 | - | 0,05 | - | 0,05 | 0,05 |
| - huile | amande douce 1,5 | tournesol 1,5 | tournesol 1,5 | tournesol 1,5 | tournesol 1,5 | tournesol 1,5 |
| - eau purifiée | q s.100g | q s.100g | q s.100g | q s.100g | q s.100g | q s.100g |
| - NaOH | q s. pH 6-7 | q s. pH 6-7 | q s. pH 6-7 | q s. pH 6-7 | q s. pH 6-7 | q s. pH 6-7 |

Pour l'ensemble des compositions selon l'invention E1, E2 et témoins EC1, EC2, EC3 et EC4 les caractéristiques de l'émulsion suivantes ont été évaluées :
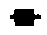 aspect macroscopique,
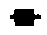 pH,
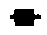 osmolalité, et
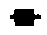 mesure de la taille des globules huileux dans le véhicule par granulométrie laser. Le volume moyen exprimé en µm est reporté pour chaque échantillon.

La tenue de l'émulsion à la centrifugation a aussi été évaluée comme test prévisionnel de stabilité. Les échantillons ont été centrifugés pendant 5 minutes à différentes vitesses et examinés visuellement afin de déterminer les modifications physiques telles que le crémage (accumulation de globules lipidiques en surface) ou le déphasage (séparation complète des phases huileuse et aqueuse).

L'exemple E1 selon l'invention a été suivi en stabilité prolongée sur 6 mois à 4°C et 40°C et sur 18 mois à 25°C. Les paramètres suivants ont été étudiés : aspects macroscopique et microscopique, pH, osmolalité, taille des globules et viscosité (à 25°C.

Les résultats obtenus sont présents dans le tableau 2 ci-après.

**Tableau 2**

| Compositions | Aspect macroscopique | pH | Osmolalité (mOsm/kg) | Taille des globules (volume moyen) | Tenue à la centrifugation | Stabilité |
|---|---|---|---|---|---|---|
| E1 | Emulsion très blanche et homogène | 6,6 | 268 | 4,7 µm | + (Pas de modification) | Pas de modification significative après 6 mois à 4°C, 25°C et 40°C pour chaque paramètre testé |
| E2 | Emulsion très blanche et homogène | 6.8 | 275 | 6,3 µm | + (Pas de modification) | - |
| EC1 | Emulsion blanche et homogène | 6,6 | 268 | 27,6 µm | - (Crémage important) | - |
| EC2 | Emulsion blanche et homogène | 5,2 | 267 | 23,5 µm | + (Pas de modification) | - |
| EC3 | Emulsion très blanche, homogène et opaque | 6,7 | 277 | 2,2 µm | + (Pas de modification) | - |
| EC4 | Emulsion très blanche, homogène et opaque | 6.5 | 281 | 2,9 µm | + (Pas de modification) | - |

Dans les conditions de fabrication selon l'invention, les exemples E1 et E2 contenant une faible quantité de tensioactif (0,03% de PVA) présentent une taille de globules lipidiques :
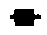 du même ordre de grandeur que les exemples comparatifs EC3 et EC4 obtenus avec une quantité de tensioactif de 1%,
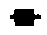 et bien inférieure à celle obtenue sans tensioactif (exemples comparatifs EC1 et EC2).

Par ailleurs, l'évaluation de la stabilité à la centrifugation révèle l'absence de modification physique pour les exemples E1 et E2 selon l'invention alors que ceux-ci contiennent peu de tensioactif. L'absence de tensioactif dans des conditions similaires (exemple comparatif EC1) conduit à un crémage de l'émulsion.

L'exemple comparatif EC2 qui ne contient pas de tensioactif n'a pas montré de modification physique lors de la centrifugation, ce qui s'explique par une viscosité plus importante de cette formule.

L'exemple E1 selon l'invention suivi pendant 6 mois à 4°C et 40°C et 18 mois à 25°C a démontré une stabilité satisfaisante pour chaque paramètre étudié.

Une étude de tolérance oculaire animale (lapin albinos) de 4 jours avec des posologies allant jusqu'à 6 instillations par jour a été réalisée sur des préparations correspondant aux exemples E1 et E2 comparativement à une condition contrôle représentée par du sérum physiologique. L'examen oculaire a été réalisé à l'aide d'un ophtalmoscope et l'évaluation de la tolérance oculaire a été faite en utilisant l'échelle d'évaluation du test de Draize tel que décrit dans la publication scientifique « Methods for the study of irritation and toxicity of substances applied topically to the skin and mucous membranes » de Draize et al. (1944), in J. pharmacol Exper Ther. 82:377.390. Les exemples E1 et E2 ont été aussi bien tolérés que la condition contrôle (sérum physiologique).

## Revendications

1. Véhicule sous forme d'émulsion huile-dans-eau (H/E) comportant au moins une phase aqueuse et au moins une phase huileuse et étant exempt d'halogénure d'ammonium quaternaire comportant des groupements alkyles en C₁₂, C₁₄, ou en C₁₆, ladite phase aqueuse comportant au moins un tensioactif hydrosoluble et au moins un agent viscosant, **caractérisé en ce que** ledit au moins un tensioactif hydrosoluble est un alcool polyvinylique et représente 0,03% en poids du poids total de véhicule.

2. Véhicule selon la revendication 1, **caractérisé en ce que** la phase huileuse se présente sous forme de globules lipidiques en dispersion dans la phase aqueuse et dont la taille est égale ou inférieure à 40 µm.

3. Véhicule selon la revendication 1 ou 2, **caractérisé en ce que** l'alcool polyvinylique (PVA) est partiellement hydrolysé.

4. Véhicule selon l'une quelconque des revendications précédentes, **caractérisé en ce que** l'agent viscosant hydrosoluble est choisi parmi les polymères acryliques, les gommes xanthanes, les carraghénanes, les alginates, la cellulose et ses dérivés.

5. Véhicule selon la revendication 4, **caractérisé en ce que** l'agent viscosant hydrosoluble est un acide polyacrylique, de préférence un acide polyacrylique réticulé.

6. Véhicule selon la revendication 4, **caractérisé en ce que** l'agent viscosant hydrosoluble est un copolymère acrylate/acrylate d'alkyle en C₁₀-C₃₀.

7. Véhicule selon l'une quelconque des revendications précédentes, **caractérisé en ce que** la phase huileuse comprend au moins une huile choisie dans le groupe constitué des huiles minérales, des huiles végétales, des huiles siliconées, des huiles de synthèse et des huiles fluorées.

8. Véhicule selon la revendication 7, **caractérisé en ce que** l'huile est une huile végétale choisie parmi les huiles de jojoba, avocat, sésame, tournesol, maïs, soja, carthame, pépins de raisin, olive, amande douce, ricin, moringa, coco, palme, bourrache, colza, germe de blé, lin, onagre, argan, calendula et coton.

9. Véhicule selon l'une quelconque des revendications précédentes, **caractérisé en ce que** la phase huileuse représente 0,5% à 5 % en poids.

10. Véhicule selon la revendication 9, **caractérisé en ce qu'**il comprend un ou plusieurs additifs choisi(s) parmi les agents conservateurs, les antioxydants, les isotonisants, les agents chélatants, les tampons, les polymères, les charges, les gélifiants hydrophiles ou lipophiles, les filtres hydrophiles, et les absorbeurs d'odeur.

11. Véhicule selon la revendication 10, **caractérisé en ce qu'**il comprend un isotonisant, de préférence la glycérine et la phase huileuse représente 0,5% à 3%, de préférence environ 1,5% en poids du poids total du véhicule, de sorte que ledit véhicule est compatible avec un usage ophtalmique.

12. Véhicule selon la revendication 10, **caractérisé en ce que** la phase huileuse représente 1 % à 5%, de préférence environ 3 % en poids du poids total du véhicule, de sorte que ledit véhicule est compatible avec un usage cosmétique et/ou dermatologique.

13. Composition ophtalmique se présentant sous forme d'une émulsion huile-dans-eau, **caractérisée en ce qu'**elle comprend :
- un véhicule tel que défini selon la revendication 11, et
- au moins un actif à usage ophtalmique.

14. Composition cosmétique et/ou dermatologique se présentant sous forme d'une émulsion huile-dans-eau, **caractérisée en ce qu'**elle comprend :
- un véhicule tel que défini selon la revendication 12, et
- au moins un actif à usage cosmétique et/ou dermatologique.

15. Procédé de préparation d'un véhicule tel que défini selon l'une quelconque des revendications 1 à 12, comprenant les étapes successives suivantes :
a) préparer une première émulsion huile-dans-eau en introduisant dans un réacteur 0,5% à 5% en poids d'au moins une huile par rapport au poids total du véhicule, puis ajouter une solution aqueuse d'alcool polyvinylique comprenant 1,5% en poids d'alcool polyvinylique en un volume tel que le rapport volumique de la phase aqueuse sur la phase huileuse est d'au moins 2/3 et préférentiellement 3/2,
b) préparation d'une solution aqueuse d'agent viscosant hydrosoluble, dont la concentration dépendra du viscosant utilisé et de l'application visée,
c) introduction de la première émulsion dans la solution aqueuse d'agent viscosant hydrosoluble, pour obtenir une deuxième émulsion comprenant une phase aqueuse et une phase huileuse, la quantité de première émulsion introduite dans la solution aqueuse d'agent viscosant hydrosoluble étant telle que l'alcool polyvinylique hydrosoluble représente 0,03% en poids du véhicule.

16. Procédé selon la revendication 15, **caractérisé en ce que** l'agent viscosant hydrosoluble est un polymère acrylique.

17. Procédé selon la revendication 15 ou 16, **caractérisé en ce que** l'on introduit, préalablement à l'étape c), une solution aqueuse d'isotonisant dans la solution aqueuse d'agent viscosant hydrosoluble, de préférence la glycérine.

## Patentansprüche

1. Mittel in Form einer Öl-in-Wasser-Emulsion (Ö/W), das wenigstens eine wässrige Phase und wenigstens eine Ölphase umfasst und frei von quartärem Ammoniumhalogenid ist, das C₁₂-, C₁₄- oder C₁₆-Alkylgruppen umfasst, wobei besagte wässrige Phase wenigstens ein wasserlösliches Tensid und wenigstens ein Viskosität verleihendes Mittel umfasst, **dadurch gekennzeichnet, dass** besagte wenigstens ein wasserlösliche Tensid Polyvinylalkohol ist und 0,03 Gew.-% des Gesamtgewichts des Mittels darstellt.

2. Mittel gemäß Anspruch 1, **dadurch gekennzeichnet, dass** die Ölphase in Form von in der wässrigen Phase dispergierten Lipidkügelchen vorliegt und deren Größe gleich oder kleiner als 40 µm ist.

3. Mittel gemäß Anspruch 1 oder 2, **dadurch gekennzeichnet, dass** der Polyvinylalkohol (PVA) partiell hydrolysiert ist.

4. Mittel gemäß einem der vorhergehenden Ansprüche, **dadurch gekennzeichnet, dass** das wasserlösliche, Viskosität verleihende Mittel aus Acrylpolymeren, Xanthangummi, Carrageen, Alginaten, Cellulose und Derivaten davon ausgewählt ist.

5. Mittel gemäß Anspruch 4, **dadurch gekennzeichnet, dass** das wasserlösliche, Viskosität verleihende Mittel eine Polyacrylsäure, vorzugsweise eine netzförmige Polyacrylsäure, ist.

6. Mittel gemäß Anspruch 4, **dadurch gekennzeichnet, dass** das wasserlösliche, Viskosität verleihende Mittel ein Acrylat/C₁₀-C₃₀-Alkylacrylat-Copolymer ist.

7. Mittel gemäß einem der vorhergehenden Ansprüche, **dadurch gekennzeichnet, dass** die Ölphase wenigstens ein Öl umfasst, das aus der Gruppe ausgewählt ist, die aus Mineralölen, Pflanzenölen, Silikonölen, Syntheseölen und fluorierten Ölen besteht.

8. Mittel gemäß Anspruch 7, **dadurch gekennzeichnet, dass** das Öl ein Pflanzenöl ist, das aus Jojoba-, Avocado-, Sesam-, Sonnenblumen-, Mais-, Sojabohnen-, Färberdistel-, Traubenkern-, Oliven-, Süßmandel-, Rizinus-, Moringa-, Kokos-, Palm-, Borretsch-, Raps-, Weizenkeim-, Lein-, Nachtkerzen-, Argan-, Calendula- und Baumwollsamenöl ausgewählt ist.

9. Mittel gemäß einem der vorhergehenden Ansprüche, **dadurch gekennzeichnet, dass** die Ölphase 0,5 Gew.-% bis 5 Gew.-% darstellt.

10. Mittel gemäß Anspruch 9, **dadurch gekennzeichnet, dass** es ein oder mehrere Additive umfasst, die aus Konservierungsmitteln, Antioxidantien, Isotonisierungsmitteln, Chelatbildnern, Puffern, Polymeren, Füllstoffen, hydrophilen oder lipophilen Gelbildnern, hydrophilen Filtern, und Geruchsabsorber ausgewählt sind.

11. Mittel gemäß Anspruch 10, **dadurch gekennzeichnet, dass** es ein Isotonisierungsmittel, vorzugsweise Glycerin, umfasst und die Ölphase 0,5 Gew.-% bis 3 Gew.-%, vorzugsweise ungefähr 1,5 Gew.-% des Gesamtgewichts des Mittels darstellt, so dass besagtes Mittel mit einer ophthalmischen Verwendung vereinbar ist.

12. Mittel gemäß Anspruch 10, **dadurch gekennzeichnet, dass** die Ölphase 1 Gew.-% bis 5 Gew.-%, vorzugsweise ungefähr 3 Gew.-% des Gesamtgewichts des Mittels darstellt, so dass besagtes Mittel mit einer kosmetischen und/oder dermatologischen Verwendung vereinbar ist.

13. Ophthalmische Zusammensetzung, die in Form einer Öl-in-Wasser-Emulsion vorliegt, **dadurch gekennzeichnet, dass** sie umfasst:
- ein Mittel wie gemäß Anspruch 11 definiert, und
- wenigstens einen Wirkstoff zur ophthalmischen Verwendung.

14. Kosmetische und/oder dermatologische Zusammensetzung, die in Form einer Öl-in-Wasser-Emulsion vorliegt, **dadurch gekennzeichnet, dass** sie umfasst:
- ein Mittel wie gemäß Anspruch 11 definiert, und
- wenigstens einen Wirkstoff zur kosmetischen und/oder dermatologischen Verwendung.

15. Verfahren zur Herstellung eines Mittels wie gemäß einem der Ansprüche 1 bis 12 definiert, umfassend die folgenden aufeinanderfolgenden Schritte:
a) Herstellen einer ersten Öl-in-Wasser-Emulsion, indem 0,5 Gew.-% bis 5 Gew.-% wenigstens eines Öls bezogen auf das Gesamtgewicht des Mittels in einen Reaktor eingebracht werden, dann Hinzufügen einer wässrigen Polyvinylalkohol-Lösung, umfassend 1,5 Gew.-% des Polyvinylalkohol in einem solchen Volumen, dass das Volumenverhältnis von wässriger Phase zu Ölphase wenigstens 2/3 und vorzugsweise 3/2 beträgt,
b) Herstellen einer wässrigen Lösung des wasserlöslichen, Viskosität verleihenden Mittels, dessen Konzentration von dem eingesetzten Viskosität verleihenden Mittel und der beabsichtigten Anwendung abhängt,
c) Einbringen der ersten Emulsion in die wässrige Lösung des wasserlöslichen, Viskosität verleihenden Mittels, um eine zweite Emulsion zu erhalten, umfassend eine wässrige Phase und eine Ölphase, wobei die Menge der ersten Emulsion, die in die wässrige Lösung des wasserlöslichen, Viskosität verleihenden Mittels eingebracht wird, so bemessen ist, dass das wasserlösliche Polyvinylalkohol 0,03 Gew.-% des Mittels darstellt.

16. Verfahren gemäß Anspruch 15, **dadurch gekennzeichnet, dass** das wasserlösliche, Viskosität verleihende Mittel ein Acrylpolymer ist.

17. Verfahren gemäß Anspruch 15 oder 16, **dadurch gekennzeichnet, dass** vor Schritt c) eine wässrige Lösung eines Isotonisierungsmittels, vorzugsweise Glycerin, in die wässrige Lösung des wasserlöslichen, Viskosität verleihenden Mittels eingebracht wird.

## Claims

1. A carrier in oil-in-water (O/W) emulsion form comprising at least one aqueous phase and at least one oily phase and being free of quaternary ammonium halide comprising C₁₂, C₁₄, or C₁₆ alkyl groups, said at least one aqueous phase comprising at least one water-soluble surfactant and at least one thickener, wherein said at least one water-soluble surfactant is a polyvinyl alcohol and represents 0.03% by weight of a total weight of the carrier.

2. The carrier according to claim 1, wherein the oily phase is provided in the form of lipid globules in dispersion in the aqueous phase and whose size is equal to or less than 40 pm.

3. The carrier according to claim 1 or 2, wherein the polyvinyl alcohol (PVA) is partially hydrolyzed.

4. The carrier according to any one of the preceding claims, wherein the water-soluble thickener is chosen among acrylic polymers, xanthan gum, carrageenans, alginates, cellulose and derivatives of same.

5. The carrier according to claim 4, wherein the water-soluble thickener is a polyacrylic acid, preferably a crosslinked polyacrylic acid.

6. The carrier according to claim 4, wherein the water-soluble thickener is a C₁₀-C₃₀ alkyl acrylate/acrylate copolymer.

7. The carrier according to any one of preceding claims, wherein the oily phase comprises at least one oil chosen from the group consisting of mineral oils, vegetable oils, silicone oils, synthetic oils and fluorinated oils.

8. The carrier according to claim 7, wherein the oil is a vegetable oil chosen among jojoba, avocado, sesame, sunflower, corn, soya, safflower, grape seed, olive, sweet almond, castor bean, moringa, coconut, palm, borage, colza, wheat germ, flax, evening primrose, argan, calendula and cotton oil.

9. The carrier according any one of preceding claims, wherein the oily phase represents 0.5% to 5% by weight.

10. The carrier according to claim 9, wherein the carrier comprises at least one or several additives selected among preservatives, antioxidants, tonicity agents, chelating agents, buffers, polymers, loads, hydrophilic or lipophilic gelling agents, hydrophilic filters, and odour absorbers.

11. The carrier according to claim 10, wherein it comprises a tonicity agent, preferably glycerin and the oily phase represents 0.5% to 3%, preferably approximately 1.5% by weight of the total weight of the carrier, so that said carrier is compatible with an ophthalmic use.

12. The carrier according to claim 10, wherein the oily phase represents 1% to 5%, preferably approximately 3% by weight of the total weight of the carrier, so that said carrier is compatible with one of a cosmetic and/or dermatological use.

13. An ophthalmic composition provided in the form of an oil-in-water emulsion, wherein it comprises:
- a carrier as defined in claim 11, and
- at least one active ingredient for ophthalmic use.

14. A cosmetic and/or dermatological composition provided in the form of an oil-in-water emulsion, wherein it comprises:
- a carrier as defined in claim 12, and
- at least one active ingredient for cosmetic and/or dermatological use.

15. A method of preparing a carrier such as defined in any one of claims 1 to 12, comprising the following steps of:
a) preparing a first oil-in-water emulsion by introducing into a reactor from 0.5% to 5% by weight of at least one oil compared to a total weight of the carrier, then adding an aqueous solution of polyvinyl alcohol comprising 1.5% by weight of polyvinyl alcohol in a volume such that a volume ratio of an aqueous phase to an oily phase is at least 2/3 and preferably 3/2,
b) preparing an aqueous solution of a water-soluble thickener whose concentration depends on the thickener and an application in question,
c) introducing the first emulsion in the aqueous solution of water-soluble thickener to obtain a second emulsion comprising the aqueous phase and the oily phase, wherein the quantity of the first emulsion introduced in the aqueous solution of water-soluble thickener is such that the water-soluble polyvinyl alcohol represents 0.03% by weight of the carrier.

16. The method according to claim 15, wherein the water-soluble thickener is an acrylic polymer.

17. The method according to claim 15 or 16, further comprising before step c), introducing an aqueous solution of tonicity agent in an aqueous solution of water-soluble thickener, preferably glycerin.
